(19) Europäisches Patentamt
European Patent Office
Office européen des brevets

(11) **EP 1 255 498 B1**

(12) **EUROPÄISCHE PATENTSCHRIFT**

(45) Veröffentlichungstag und Bekanntmachung des Hinweises auf die Patenterteilung:
**23.11.2005 Patentblatt 2005/47**

(51) Int Cl.⁷: **A61B 17/80**

(21) Anmeldenummer: 00900486.2

(22) Anmeldetag: **27.01.2000**

(86) Internationale Anmeldenummer:
**PCT/CH2000/000037**

(87) Internationale Veröffentlichungsnummer:
**WO 2001/054601 (02.08.2001 Gazette 2001/31)**

(54) **KNOCHENPLATTE**

BONE PLATE

PLAQUE POUR OSTEOSYNTHESE

(84) Benannte Vertragsstaaten:
**AT BE CH CY DE DK ES FI FR GB GR IE IT LI LU MC NL PT SE**

(43) Veröffentlichungstag der Anmeldung:
**13.11.2002 Patentblatt 2002/46**

(73) Patentinhaber: **Synthes AG Chur**
**7002 Chur (CH)**

(72) Erfinder: **FRIGG, Robert**
**CH-2544 Bettlach (CH)**

(74) Vertreter: **Lusuardi, Werther Giovanni et al**
**Dr. Lusuardi AG,**
**Kreuzbühlstrasse 8**
**8008 Zürich (CH)**

(56) Entgegenhaltungen:
**US-A- 3 716 050       US-A- 4 408 601**
**US-A- 5 709 686**

**Beschreibung**

Die Erfindung betrifft eine Knochenplatte gemäss dem Oberbegriff des Patentanspruchs 1 sowie eine Fixationsvorrichtung mit einer solchen Knochenplatte gemäss dem Oberbegriff des Patentanspruchs 20.

Grundsätzlich kennt man zwei Arten der mit Knochenplatten erfolgenden Osteosynthese.

Die erste betrifft die "Rigide Osteosynthese". Die rigide Osteosynthese wird bei der Versorgung von Gelenksfrakturen, einfachen Schaftfrakturen (wenn keine Nagelung vorgenommen werden kann) sowie bei Osteotomien angewandt. Neben der anatomischen Repositionsmöglichkeit unterstützt der Knochen selber die Stabilität der Osteosynthese, was zu einer früheren und schmerzfreieren Belastung der Extremität führt. Vorteile einer stabilen Frakturversorgung können auch dort beobachtet werden, wo die Knochendurchblutung durch das Trauma bedingt stark vermindert ist. Bei der Versorgung von "non-unions" oder bei vorhandener Infektion, muss die Fraktur stabil versorgt werden, um eine Knochenheilung zu ermöglichen und um die Infektion nicht durch die Instabilität im Frakturspalt zusätzlich zu reizen.

Die zweite betrifft die "Flexible Osteosynthese". Die grössten Vorteile der flexiblen (biologischen) Osteosynthese sind bei der Versorgung von Trümmerfrakturen im Schaftbereich von Röhrenknochen zu sehen. Bei diesen Frakturen ist das Ziel die Länge des Knochens, sowie die Knochenenden (Gelenke) in korrekter Lage zueinander zu halten. Die Frakturzone wird dabei nicht direkt fixiert oder manipuliert, was die Durchblutung dieser Zone nicht zusätzlich beeinträchtigt. Die Knochenplatten funktionieren ähnlich einem Verriegelungs-Marknagel, der nur in den Metaphysen verankert ist.

Betrachtet man nun diese beiden Extreme der Plattenosteosynthese, erkennt man wie weit diese auseinander liegen. Da sich nicht immer alle Frakturen in eine der beiden oben genannten Osteosynthese-Arten einteilen lassen, muss der Chirurg oft Kompromisse eingehen, da ihm kein Implantat zur Verfügung steht, welches ihm erlaubt beide Methoden kompromisslos zu kombinieren. Eine solche Kombination wäre z.B. dann sinnvoll, wenn eine Gelenksfraktur mit Zugschrauben durch die Knochenplatte komprimiert werden kann und der gesamte Gelenksteil über einen internen Fixateur, mit winkelstabilen Schrauben, zur Diaphyse verbunden wird. Ein weiter Anwendungsfall wäre z.B. bei porotischem Knochen , wo eine Knochenplatte mit axial und winkelstabilen Schrauben im metaphysären Fragment verankert werden kann, wobei im diaphysären Bereich eine stabile Verplattung vorgenommen werden kann, mit der Unterstützung einer Plattenzugschraube durch die Fraktur. Dank dieser Versorgung kann eine primäre Frakturstabilisierung erreicht werden.

Diese Situation hat dazu geführt, dass man Knochenimplantate für beide Arten der Osteosynthese entwickelt und auf den Markt gebracht hat. Beide Implantategruppen sind für ihre jeweilige Methode optimal ausgelegt. Der Nachteil dieser beiden System liegt somit in ihrer fehlenden Kombinationsmöglichkeit.

Aus der US-A 4,408,601 WENK ist eine Knochenplatte bekannt, deren Plattenloch eine kreisförmige Partie mit einer Vertiefung aufweist, deren Oberfläche glatt ausgebildet ist. Der in die Vertiefung einzuführende - ebenfalls glatte - sphärische Schraubenkopf soll auf der glatten Oberfläche der Vertiefung gleiten können.

Aus der US 5,709,686 TALOS ET AL. ist eine Kombinationsplatte bekannt, bei welcher ein zylindrisches Gewinde in der mittleren Partie des Langlochs angebracht ist. Die Nachteile dieser bekannten Platte sind aber die folgenden:

1) Die mittständige Lage des Gewindes im Langloch der Platte beschränkt den Bereich des Gewindes auf 60° bis 179°;

2) Die mittständige Lage des Gewindes im Langloch (Spannloch) der Platte weist die Gefahr auf, dass sich die seitlichen Stege des Langlochs aufweiten können;

3) Wegen der zylindrischen Form des Gewindes muss ein speziell ausgebildeter Schraubenkopf verwendet werden, der sich beim Eindrehen auf der Plattenoberfläche abstützen kann.

Hier will die Erfindung Abhilfe schaffen. Der Erfindung liegt das Problem zugrunde, eine Knochenplatte zu schaffen, welche beide Osteosynthesearten in sich kombiniert, ohne jedoch Einschränkungen bei den beiden reinen Plattenversorgungsmethoden zur Folge zu haben. Sie soll demnach die kompromisslose Verwendung der Platte als Kompressionsplatte und als sogenannter Fixateur interne erlauben.

Die Erfindung löst die gestellte Aufgabe mit einer Knochenplatte, welche die Merkmale des Anspruchs 1 aufweist.

Als Langloch im Sinne der Erfindung soll ein Loch gelten, dessen Durchmesser in Richtung der Plattenlängsachse gemessen grösser ist als der Durchmesser dieses Loches senkrecht zur Plattenlängsachse gemessen. Ein solches Langloch kann somit oval, ellipsenförmig oder auch rechteckig ausgebildet sein oder eine Kombination solcher Formen beinhalten; lediglich kreisrunde Löcher sind von dieser Definition ausgeschlossen.

Die dreidimensionalen Strukturierung kann in einer einzigen - zur Oberseite der Knochenplatte parallelen - Ebene angeordnet sein oder in mehreren zur Oberseite parallelen Ebenen angeordnet sein.

Der Durchmesser d des kreisförmigen Loches ist vorzugsweise kleiner als die kurze Achse b des Langlochs des Kombinationsloches. Typischerweise ist d um 5 bis 25 % kleiner als b.

Die Anwendung der Platte als Fixateur interne führt zu einer stark erhöhten mechanischen Beanspruchung des Platten-Schrauben-Interface, da die Platte nicht auf den Knochen gedrückt wird und so die Knochenfraktur mittels Reibung zwischen Platte und Knochen fixiert wird. Dieser mechanischen Mehrbelastung wird bei einer bevorzugten Ausführungsform dadurch Rechnung getragen, dass sich das Gewinde im Langloch über einen Bereich von mindestens 180° erstreckt und somit das Schraubenkopf-Gewinde um mindestens diesen Winkelbereich umschliesst. Bei dünnen Knochenplatten ist dieser Umstand von besonderer Bedeutung.

Eine bevorzugte Weiterbildung besteht darin, dass sich die dreidimensionale Strukturierung (z.B. in Form eines Innengewindes) im Kombinationsloch gegen die Unterseite der Knochenplatte hin - vorzugsweise konisch - verjüngt. Sie hat den Vorteil, dass die Fixation der Schraube durch das konische Gewinde des Plattenlochs und das korrespondierende konische Gewinde des verwendeten Schraubenkopfes erfolgt. Diese Art der Fixation ist besonders wichtig, wenn man selbstbohrende Schrauben verwenden will. Dank des konischen Gewindes im Kopfbereich der Schraube, kann der Einbringvorgang der Schraube in den Knochen, unabhängig von der Platte erfolgen. Erst wenn der Gewindekonus des Schraubenkopfes in das Innengewinde des Langlochs der Platte eindringt, wird die Schraube blockiert. Trotz unterschiedlicher Gewindeanfänge im Plattenloch-Konus und im Knochen zentriert sich das konische Schraubenkopfgewinde im Gewindekonus der Platte.

Beim Festziehen des konischen Gewindes entstehen radiale Kräfte im Plattenloch. Um diese ausreichend aufzunehmen, muss das konische Plattenloch eine ausreichende Stabilität aufweisen.

Das bei einer bevorzugten Ausführungsform gegen die Unterseite der Knochenplatte hin sich konisch verjüngende Innengewinde weist zweckmässigerweise einen Konuswinkel von 5 - 20° auf, typischerweise von 10° auf.

Bei einer weiteren bevorzugten Ausbildung der Erfindung ist das Innengewinde - in Richtung der Plattenlängsachse gesehen - an einem der beiden Enden des Langlochs angebracht. Diese Position erlaubt es konstruktiv einen vergrösserten Gewindebereich zu realisieren, der sich z.B. von 190° bis 280°, vorzugsweise von 200° bis 250° des von ihm gebildeten geometrischen Körpers erstreckt.

Falls das Langloch konisch ausgebildet ist ergibt die Messung der Ausdehnung des Innengewindes an der Unterseite, bzw. an der Oberseite der Platte verschieden grosse Werte. Bei einer Messung an der Oberseite sollte sich der Bereich des Gewindes vorzugsweise über 180° bis 230° erstrecken; bei einer Messung an der Unterseite über 200° bis 270°.

Bei einer weiteren bevorzugten Ausführungsform ist das endständige, konische Gewinde im Langloch (Spannloch) an jenem Ende angebracht, welches näher zur Plattenmitte liegt. Dies hat den Vorteil, dass die Spannfunktion der Plattenspannlöcher nicht beeinträchtigt wird.

Bei einer weiteren bevorzugten Ausführungsform weist das Kombinationsloch in seinem oberen, der Oberseite zugewandten Teil, eine konkave, vorzugsweise sphärische Erweiterung zur Aufnahme einer Knochenschraube mit einem kugeligen Kopf auf. Der kugelförmige Schraubenkopf einer herkömmlichen Knochen schraube findet in dieser konkaven, sphärischen Erweiterung einen optimalen Sitz. Dies vor allem dann, wenn die Knochenschraube exzentrisch eingebracht wurde, was zur Erreichung einer Frakturkompression nötig ist.

Bei einer weiteren bevorzugten Ausführungsform ist die Unterseite konkav ausgebildet ist. Durch die konkave Unterseite der Platte, passt sich diese besser an den runden Knochenquerschnitt der Tibia, des Femur, des Humerus und der Unterarmknochen an. Durch die konkave Ausführungsform des Loches an der Plattenunterseite, kann eine herkömmliche Knochenschraube schräg durch das Plattenloch eingesetzt werden. Das kann vor allem für das Fassen eines kleinen Knochenfragments wichtig sein, das an die Platte herangezogen werden muss.

Bei einer weiteren bevorzugten Ausführungsform erstreckt sich das Innengewinde über die gesamte Höhe der Knochenplatte von der Unterseite bis zur Oberseite, um eine möglichst hohe Stabilität zu erreichen.

Bei einer weiteren bevorzugten Ausführungsform erweitert sich das Kombinationsloch in seinem unstrukturierten Sektor, in seinem unteren, der Unterseite zugewandten Teil, so dass eine Auslenkung der Knochenschraube möglich wird.

Eine weitere Ausführungsform umfasst neben der erfindungsgemässen Knochenplatte zusätzlich mindestens eine Knochen schraube; sie kann eine zur dreidimensionalen Strukturierung korrespondierende, am Schraubenkopf angebrachte Strukturierung, z. B. in Form eines Aussengewindes aufweisen, welche vorzugsweise selbstschneidend und/oder selbstbohrend ausgebildet ist.

Bei der Verwendung der Knochenplatte als Kompressionsplatte, wird die Spannlochgeometrie der Plattenbohrung, durch das endständige, konische Gewindeloch, nicht negativ beeinflusst. Der Vorteil der konischen Ausführung des Gewindeloches ist das plattenunabhängige Einbringen der Schraube in den Knochen, wobei sich die Schraube erst beim Festziehen mit der Platte, über einen entsprechend konisch ausgebildeten, gewindeten Schraubenkopf, verbindet. Das ist vor allem bei der Verwendung von selbstbohrenden, selbstschneidenden Schrauben vorteilhaft.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine schematische Darstellung des aus einem kreisförmigen Loch und einem Langloch bestehenden Kombinationsloches;

Fig. 2 eine Aufsicht auf eine erfindungsgemässe Knochenplatte mit einem Kombinationsloch mit dreidimensionaler Strukturierung;

Fig. 3 einen Längsschnitt durch das kreisrunde Loch des Kombinationsloches von Fig. 2; und

Fig. 4 eine perspektivische Darstellung durch die erfindungsgemässe Knochenplatte mit einer im Kombinationsloch mit integriertem Gewinde eingesetzten Knochenschraube.

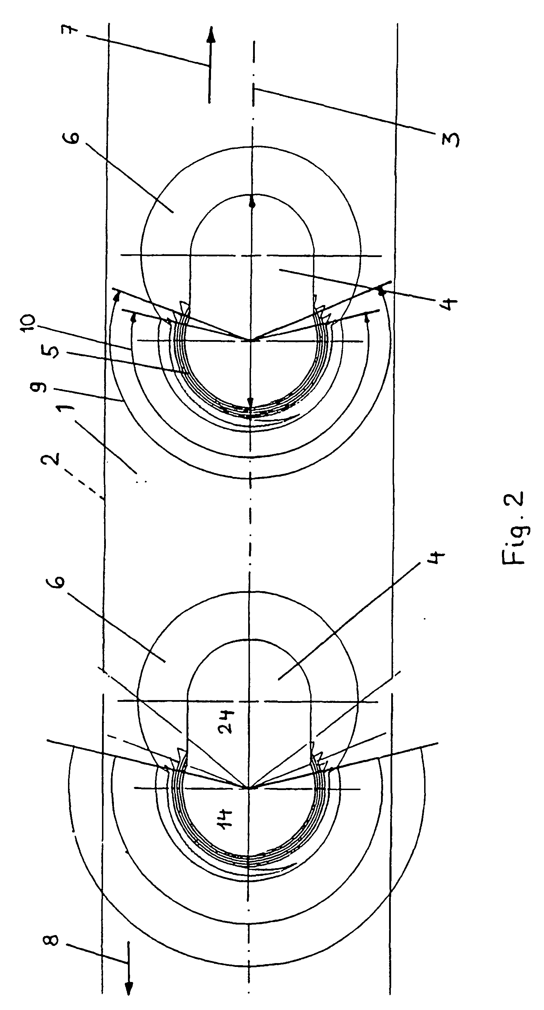

Die in Fig. 2 dargestellte erfindungsgemässe Knochenplatte besitzt eine Oberseite 1, eine für den Knochenkontakt bestimmte Unterseite 2 sowie zwei die Oberseite 1 mit der Unterseite 2 verbindenden, entlang der Plattenlängsachse 3 angeordneten Löchern 4 für die Aufnahme von Knochenschrauben 11. Der Pfeil 7 zeigt die Richtung zum einem Ende der Knochenplatte währenddem der Pfeil 8 die Richtung zur Plattenmitte anzeigt.

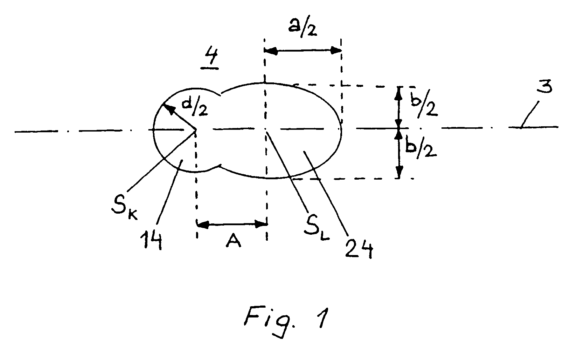

Der Durchmesser des näher zur Plattenmitte gelegenen Lochs 4 ist in Richtung der Plattenlängsachse 3 gemessen grösser als der Durchmesser dieses Loches senkrecht zur Plattenlängsachse 3 gemessen. Wie in Fig. 1 schematisch dargestellt besteht das Loch 4 aus einem kreisförmigen Loch 14 mit dem Durchmesser d und dem Symmetriezentrum $S_k$ sowie einem Langloch 24 mit dem Symmetriezentrum $S_l$, welche sich beide überlappen. Das Langloch 24 besitzt eine, in Richtung der Plattenlängsachse 3 verlaufende, lange Achse a und eine senkrecht dazu verlaufenden, kurze Achse b, wobei der Abstand A zwischen den Symmetriezentren $S_k$ und $S_l$ kleiner ist als die Summe von d/2 + a/2. Beide Symmetriezentren liegen auf der Plattenlängsachse 3 oder zumindest in deren Nähe.

In seinem oberen, der Oberseite 1 zugewandten Teil weist das Langloch 24, eine konkave, vorzugsweise sphärische Erweiterung 6 zur Aufnahme einer Knochenschraube mit einem kugeligen Kopf auf.

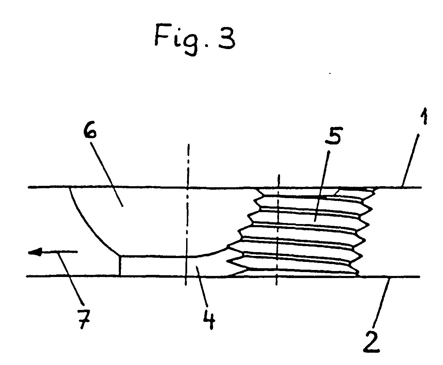

Wie in Fig. 3 dargestellt erstreckt sich die dreidimensionale Strukturierung 5 in Form eines Innengewindes 5 des näher zum Plattenende liegenden Loches 4 über die gesamte Höhe der Knochenplatte von der Oberseite 1 bis zur Unterseite 2.

Bei der in den Fig. 2 und 3 dargestellten, bevorzugten Ausführungsform der Erfindung ist das Innengewinde an demjenigen Ende des Langlochs 24 angebracht, welches näher zur Plattenmitte gelegen ist. Das Innengewinde erstreckt sich an der Unterseite 2 gemessen - wie durch den Kreisbogen 9 angedeutet - über einen Bereich von 256° und an der Oberseite 1 gemessen - wie durch den Kreisbogen 10 angedeutet - über einen Bereich von 223°.

Je nach Durchmesser des Innengewindes 5 ergeben sich folgende bevorzugte Parameter:

| Durchmesser des Gewindes | 2,4 mm | 3,5 mm | 5,0 mm |
|---|---|---|---|
| zweigängiges Gewinde | JA | JA | JA |
| Steigung des Gewindes | 0,6 | 0,8 | 1,0 |
| Tiefe des Gewindes (= halbe Differenz zwischen Aussen- und Innendurchmesser) | 0,175 | 0,2295 | 0,2810 |
| Winkelbereich (an Oberseite) | 200° | 200° | 190° |
| Winkelbereich (an Unterseite) | 260° | 240° | 250° |

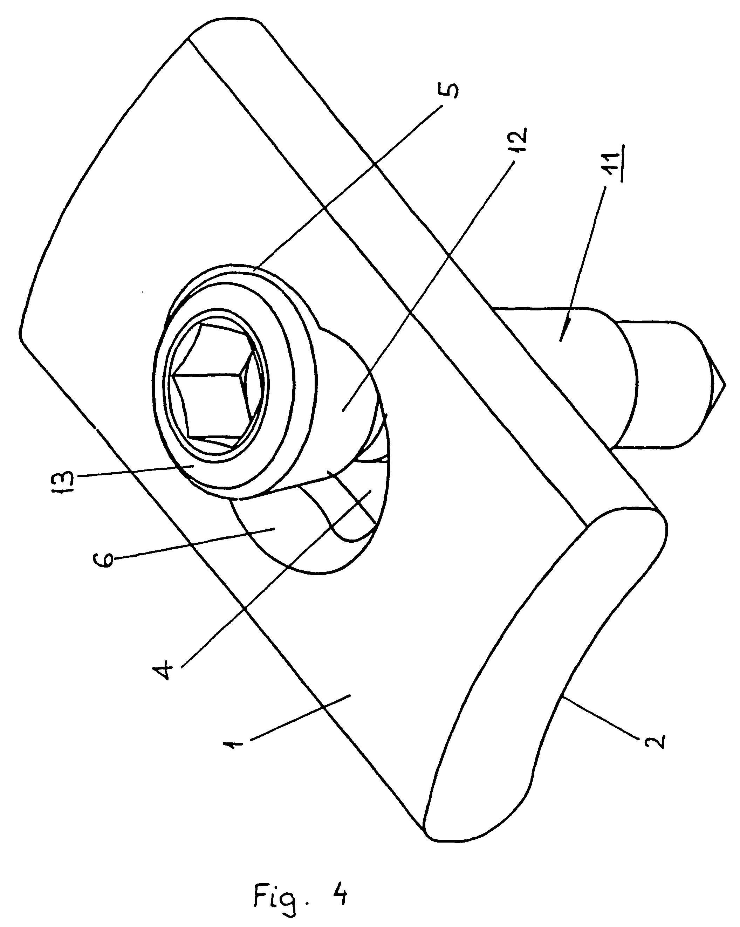

In Fig. 4 ist eine Fixationsvorrichtung mit einer Knochenplatte gemäss Fig. 2 dargestellt, welche eine Knochenschraube 11 mit einem zum Innengewinde der Knochenplatte korrespondierenden, am Schraubenkopf 13 angebrachten Aussengewinde 12 umfasst. Die Knochenschraube 11 ist zweckmässigerweise selbstbohrend und/oder selbstschneidend ausgebildet.

## Patentansprüche

1. Knochenplatte mit einer Oberseite (1), einer für den Knochenkontakt bestimmten Unterseite (2) sowie mehreren die Ober- mit der Unterseite (1;2) verbindenden, entlang der Plattenlängsachse (3) angeordneten Löchern (4) für die Aufnahme von Knochenschrauben (11), wobei

A) mindestens eines der Löcher (4) aus einer Kombination eines

  a) kreisförmigen Loches (14) mit dem Durchmesser d und dem Symmetriezentrum $S_k$ mit
  b) einem Langloch (24) mit dem Symmetriezentrum $S_l$ besteht, welches eine, in Richtung der Plattenlängsachse verlaufende, lange Achse a und eine senkrecht dazu verlaufenden, kurze Achse b besitzt, wobei

B) der Abstand A zwischen den Symmetriezentren $S_k$ und $S_l$ kleiner als die Summe von d/2 + a/2 aber nicht gleich Null ist und die beiden Symmetriezentren im Bereich der Plattenlängsachse (3) angeordnet sind; und **dadurch gekennzeichnet, dass**
C) das kreisförmige Loch (14) mit einer dreidimensionalen Strukturierung (5) versehen ist, welche in Form eines Innengewindes oder einer peripheren Lamelle oder Lippe vorliegt.

2. Knochenplatte nach Anspruch 1, **dadurch gekennzeichnet, dass** das Langloch (24) als Spannloch ausgebildet ist.

3. Knochenplatte nach Anspruch 1 oder 2, **dadurch gekennzeichnet, dass** die dreidimensionale Strukturierung (5) in einer zur Oberseite (1) parallelen Ebene angeordnet ist.

4. Knochenplatte nach Anspruch 1 oder 2, **dadurch gekennzeichnet, dass** die dreidimensionale Strukturierung (5) in mehreren zur Oberseite (1) parallelen Ebenen angeordnet ist.

5. Knochenplatte nach einem der Ansprüche 1 bis 4, **dadurch gekennzeichnet, dass** der Durchmesser d des kreisförmigen Loches (14) kleiner ist als die kurze Achse b des Langlochs (24), vorzugsweise 5 bis 25 % kleiner.

6. Knochenplatte nach einem der Ansprüche 1 bis 5, **dadurch gekennzeichnet, dass** sich die dreidimensionale Strukturierung (5) über mindestens 180° des kreisförmigen Loches (14) erstreckt.

7. Knochenplatte nach Anspruch 6, **dadurch gekennzeichnet, dass** sich die dreidimensionale Strukturierung (5) über 190° bis 280°, vorzugsweise über 200° bis 250° des kreisförmigen Loches (14) erstreckt.

8. Knochenplatte nach Anspruch 6 oder 7, **dadurch gekennzeichnet, dass** sich die dreidimensionale Strukturierung (5) - an der Oberseite (1) gemessen - über 180° bis 230° erstreckt und - an der Unterseite (2) gemessen - über 200° bis 270° erstreckt.

9. Knochenplatte nach einem der Ansprüche 1 bis 8, **dadurch gekennzeichnet, dass** sich die kombinierten Löcher (14,24) von der Oberseite (1) zur Unterseite (2) hin verjüngen.

10. Knochenplatte nach einem der Ansprüche 1 bis 9, **dadurch gekennzeichnet, dass** die dreidimensionale Strukturierung (5) - in Richtung der Plattenlängsachse (3) gesehen - an demjenigen Ende der kombinierten Löcher (14,24) angebracht ist, welches näher zur Plattenmitte (8) ausgerichtet ist.

11. Knochenplatte nach einem der Ansprüche 1 bis 10, **dadurch gekennzeichnet, dass** das Langloch (24) in seinem oberen, der Oberseite (1) zugewandten Teil, eine konkave, vorzugsweise sphärische Erweiterung (6) zur Aufnahme einer Knochenschraube (11) mit einem kugeligen Kopf (13) aufweist.

12. Knochenplatte nach einem der Ansprüche 1 bis 11, **dadurch gekennzeichnet, dass** die Unterseite (1) konkav oder planar ausgebildet ist.

13. Knochenplatte nach einem der Ansprüche 1 bis 12, **dadurch gekennzeichnet, dass** sich die dreidimensionale Strukturierung (5) über die gesamte Höhe der Knochenplatte von der Oberseite (1) bis zur Unterseite (2) erstreckt.

14. Knochenplatte nach einem der Ansprüche 1 bis 13, **dadurch gekennzeichnet, dass** sich die kombinierten Löcher (14,24) in ihrem unstrukturierten Sektor, in ihrem unteren, der Unterseite (2) zugewandten Teil erweitern.

15. Knochenplatte nach einem der Ansprüche 1 bis 14, **dadurch gekennzeichnet, dass** der Abstand A zwischen den Symmetriezentren $S_k$ und $S_l$ der Bedingung:

$$0,5 \, (d/2 + a/2) < A < 1,0 \, (d/2 + a/2)$$

gehorcht.

16. Knochenplatte nach einem der Ansprüche 1 bis 15, **dadurch gekennzeichnet, dass** die Achse mindestens eines der Löcher (4) einen Winkel von 70 - 110° zu einer zur Oberseite (1) parallelen Ebene einschliesst.

17. Knochenplatte nach einem der Ansprüche 1 bis 16, **dadurch gekennzeichnet, dass** sie aus mehreren Abschnitten besteht, so dass vorzugsweise eine L-förmige oder T-förmige Form der Platte resultiert, und die Plattenlängsachse (3) der einzelnen Abschnitte einen stumpfen oder spitzen Winkel untereinander einschliessen.

18. Knochenplatte nach Anspruch 17, **dadurch gekennzeichnet, dass** mindestens zwei Abschnitte der Knochenplatte in verschiedenen Ebenen angeordnet sind.

19. Knochenplatte nach Anspruch 17 oder 18, **dadurch gekennzeichnet, dass** im längsten Abschnitt der Knochenplatte die dreidimensionale Strukturierung (5) - in Richtung der Plattenlängsachse (3) dieses längsten Abschnittes gesehen - an demjenigen Ende der kombinierten Löcher (14,24) angebracht ist, welches näher zum sich anschliessenden kürzeren Abschnitt der Knochenplatte ausgerichtet ist.

20. Fixationsvorrichtung mit einer Knochenplatte gemäss einem der Ansprüche 1 bis 19, **dadurch gekennzeichnet, dass** sie zusätzlich mindestens eine Knochenschraube (11) umfasst.

21. Fixationsvorrichtung nach Anspruch 20, **dadurch gekennzeichnet, dass** die Knochenschraube (11) mit einer zur dreidimensionalen Strukturierung (5) korrespondierenden, am Schraubenkopf (13) angebrachten dreidimensionalen Strukturierung (12), vorzugsweise in Form eines Aussengewindes umfasst.

22. Fixationsvorrichtung nach Anspruch 20 oder 21, **dadurch gekennzeichnet, dass** die Knochenschraube (11) selbstschneidend und/oder selbstbohrend ausgebildet ist.

**Claims**

1. A bone plate with a top surface (1), a bottom surface (2) for contact with the bone, and a plurality of holes (4) situated along the longitudinal axis (3) of the plate and connecting the top surface (1) and the bottom surface (2) for receiving bone screws (11), whereby

   A) at least one of the holes (4) consists of a combination of

      a) a circular hole (14) with a diameter d and a centre of symmetry $S_k$ and
      b) an elongate hole (24) with a centre of symmetry $S_l$, having a long axis a extending in the direction of the longitudinal axis of the plate, and a short axis b extending vertically thereto, whereby

   B) the distance A between the centres of symmetry $S_k$ and $S_l$ being shorter than the sum d/2 + a/2 but not equal to zero and both centres of symmetry being situated in the region of the longitudinal axis (3) of the plate; and
   **characterized in that**
   C) the circular hole (14) is provided with a three-dimensional structure (5) which is in the form of an internal screw thread or a peripheral lamella or lip.

2. A bone plate as claimed in claim 1, **characterised in that** the enlongate hole (24) is configured as a tension hole.

3. A bone plate as claimed in claim 2, **characterised in that** the three-dimensional structure (5) is arranged in a plane parallel to the top surface (1).

4. A bone plate as claimed in claim 2, **characterised in that** the three-dimensional structure (5) is arranged in a plurality of planes parallel to the top surface (1).

**5.** A bone plate as claimed in any of the claims 1 to 4, **characterised in that** the diameter d of the circular hole (14) is smaller, preferably by 5 to 25%, than the short axis b of the elongate hole (24).

**6.** A bone plate as claimed in any of the claims 1 to 5, **characterised in that** the three-dimensional structure (5) extends over an angle of at least 180° of the circular hole (14).

**7.** A bone plate as claimed in claim 6, **characterised in that** the three-dimensional structure (5) extends over an angle of between 190° and 280°, preferably between 200° and 250°, of the circular hole (14).

**8.** A bone plate as claimed in claim 6 or 7, **characterised in that** the three-dimensional structure (5) - measured on the top surface (1) - extends over an angle of between 180° and 230° and - measured on the bottom surface (2) - over an angle of between 200° and 270°.

**9.** A bone plate as claimed in any of the claims 1 to 8, **characterised in that** the combined holes (14, 24) have a tapered form from the top surface (1) to the bottom surface (2).

**10.** A bone plate as claimed in any of the claims 1 to 9, **characterised in that** the three-dimensional structure (5) - considered in the direction of the longitudinal axis (3) of the plate - is formed in the end portion of the combined holes (14,24) which is situated closer to the centre (8) of the plate.

**11.** A bone plate as claimed in any of the claims 1 to 10, **characterised in that** the elongate hole (24) has in its upper portion, facing the top surface (1), a concave, preferably spherical enlargement (6) for receiving a bone screw (11) with a spherical screw head (13).

**12.** A bone plate as claimed in any of the claims 1 to 11, **characterised in that** the bottom surface (1) has a concave or planar shape.

**13.** A bone plate as claimed in any of the claims 1 to 12, **characterised in that** the three-dimensional structure (5) extends over the entire thickness of the bone plate, from the top surface (1) to the bottom surface (2).

**14.** A bone plate as claimed in any of the claims 1 to 13, **characterised in that** the combined holes (14,24) have an enlargement in the unstructured portion of their lower part, facing the bottom surface (2).

**15.** A bone plate as claimed in any of the claims 1 to 14, **characterised in that** the distance A between the centres of symmetry $S_k$ and $S_l$ fulfils the following condition:

$$0,5 \ (d/2 + a/2) < A < 1,0 \ (d/2 + a/2).$$

**16.** A bone plate as claimed in any of the claims 1 to 15, **characterised in that** the axis of at least one of the holes (4) forms an angle of between 70° and 110° relative to a plane parallel to the top surface (1).

**17.** A bone plate as claimed in any of the claims 1 to 16, **characterised in that** said plate consists of a plurality of sections which preferably result in an L-shaped or T-shaped form of the plate, the longitudinal axes (3) of the individual plate sections forming an acute or an obtuse angle between each other.

**18.** A bone plate as claimed in claim 17, **characterised in that** at least two sections of the bone plate are arranged in different planes.

**19.** A bone plate as claimed in claim 17 or 18, **characterised in that** the three-dimensional structure (5) of the longest section of the bone plate - considered in the direction of the longitudinal axis (3) of this longest section - is formed in the end portion of the combined holes (14,24) which is situated closer to the adjacent, shorter section of the bone plate.

**20.** A fixation device including a bone plate as claimed in any of the claims 1 to 19, **characterised in that** said plate comprises in addition at least one bone screw (11).

**21.** A fixation device as claimed in claim 20, **characterised in that** the bone screw (11) comprises a three-dimensionally structured portion (12) - preferably in the form of an external screw thread - formed in the screw head (13), which corresponds to the three-dimensionally structured portion (5).

22. A fixation device as claimed in claim 20 or 21, **characterised in that** the bone screw (11) is a self-tapping and/or self-drilling bone screw.

## Revendications

1. Plaque d'ostéosynthèse comprenant une face supérieure (1), une face inférieure (2) destinée à être en contact avec l'os ainsi que plusieurs trous (4) reliant la face supérieure (1) à la face inférieure (2), disposés le long de l'axe longitudinal (3) de la plaque et destinés à recevoir des vis d'ostéosynthèse (11),

   A) au moins un des trous (4) présentant une combinaison entre

   a) un trou circulaire (14) présentant le diamètre d et le centre de symétrie $S_k$
   et
   b) un trou oblong (24) présentant le centre de symétrie $S_l$ et qui possède un axe long a s'étendant dans le sens de l'axe longitudinal (3) de la plaque et un axe court b s'étendant perpendiculairement à celui-ci,

   B) la distance A entre les centres de symétrie $S_k$ et $S_l$ étant inférieure à la somme d/2 + a/2 mais n'étant pas égale à zéro, et les deux centres de symétrie étant situés dans la zone de l'axe longitudinal (3) de la plaque; et **caractérisée en ce que**
   C) le trou circulaire (14) est pourvu d'une structuration tridimensionnelle (5) réalisée sous la forme d'un filet intérieur ou d'une lamelle ou lèvre périphérique.

2. Plaque d'ostéosynthèse selon la revendication 1, **caractérisée en ce que** le trou oblong (24) est réalisé sous forme de trou de serrage.

3. Plaque d'ostéosynthèse selon la revendication 1 ou 2, **caractérisée en ce que** la structuration tridimensionnelle (5) est disposée dans un plan parallèle à la face supérieure (1).

4. Plaque d'ostéosynthèse selon la revendication 1 ou 2, **caractérisée en ce que** la structuration tridimensionnelle (5) est disposée dans plusieurs plans parallèles à la face supérieure (1).

5. Plaque d'ostéosynthèse selon l'une des revendications 1 à 4, **caractérisée en ce que** le diamètre d du trou circulaire (14) est inférieur à l'axe court b du trou oblong (24), et ce de préférence de 5 à 25%.

6. Plaque d'ostéosynthèse selon l'une des revendications 1 à 5, **caractérisée en ce que** la structuration tridimensionnelle (5) s'étend sur au moins 180° du trou circulaire (14).

7. Plaque d'ostéosynthèse selon la revendication 6, **caractérisée en ce que** la structuration tridimensionnelle (5) s'étend sur une zone comprise entre 190° et 280°, de préférence entre 200° et 250° du trou circulaire (14).

8. Plaque d'ostéosynthèse selon la revendication 6 ou 7, **caractérisé en ce que** la structuration tridimensionnelle (5) - mesurée au niveau de la face supérieure (1) - s'étend sur une zone comprise entre 180° et 230° et - mesurée au niveau de la face inférieure (2) - sur une zone comprise entre 200° et 270°.

9. Plaque d'ostéosynthèse selon l'une des revendications 1 à 8, **caractérisée en ce que** les trous combinés (14,24) vont en s'amincissant de manière conique depuis la face supérieure (1) vers la face inférieure (2).

10. Plaque d'ostéosynthèse selon l'une des revendications 1 à 9, **caractérisée en ce que** la structuration tridimensionnelle (5) - vue dans le sens de l'axe longitudinal (3) de la plaque - est réalisée à l'extrémité des trous combinés (14,24) qui est située plus près du centre (8) de la plaque.

11. Plaque d'ostéosynthèse selon l'une des revendications 1 à 10, **caractérisée en ce** le trou oblong (24) présente, dans sa partie supérieure tournée vers la face supérieure (1), un élargissement concave (6), de préférence sphérique, destiné à recevoir une vis d'ostéosynthèse (11) comprenant une tête sphérique (13).

**12.** Plaque d'ostéosynthèse selon l'une des revendications 1 à 11, **caractérisée en ce que** la face inférieure (2) est réalisée de manière concave ou planaire.

**13.** Plaque d'ostéosynthèse selon l'une des revendications 1 à 12, **caractérisée en ce que** la structuration tridimensionnelle (5) s'étend sur l'ensemble de la hauteur de la plaque d'ostéosynthèse, à savoir depuis la face supérieure (1) jusqu'à la face inférieure (2).

**14.** Plaque d'ostéosynthèse selon l'une des revendications 1 à 13, **caractérisée en ce que** les trous combinés s'élargissent dans leur zone non structurée, à savoir dans leur partie inférieure tournée vers la face inférieure (2).

**15.** Plaque d'ostéosynthèse selon l'une des revendications 1 à 14, **caractérisée en ce que** la distance A entre les centres de symétrie $S_k$ et $S_i$ obéit à la condition suivante :

$$0,5 \, (d/2 + a/2) < A < 1,0 \, (d/2 + a/2).$$

**16.** Plaque d'ostéosynthèse selon l'une des revendications 1 à 15, **caractérisée en ce que** l'axe d'au moins un des trous (4) forme un angle compris entre 70° et 110° par rapport à un plan parallèle à la face supérieure (1).

**17.** Plaque d'ostéosynthèse selon l'une des revendications 1 à 16, **caractérisée en ce qu'**elle se compose de plusieurs parties, de sorte que ladite plaque présente de préférence une forme en L ou en T, et que les axes longitudinaux (3) des différentes parties constituant la plaque forment entre eux un angle obtus ou un angle aigu.

**18.** Plaque d'ostéosynthèse selon la revendication 17, **caractérisée en ce qu'**au moins deux parties de la plaque d'ostéosynthèse sont disposées dans des plans différents.

**19.** Plaque d'ostéosynthèse selon la revendication 17 ou 18, **caractérisée en ce que**, dans la partie la plus longue de la plaque d'ostéosynthèse, la structuration tridimensionnelle (5) - vue dans le sens de l'axe longitudinal (3) de cette partie la plus longue de la plaque - est réalisée sur l'extrémité des trous combinés (14,24) qui est située plus près de la partie plus courte de la plaque d'ostéosynthèse, laquelle se situe dans le prolongement de cette dernière.

**20.** Dispositif de fixation comportant une plaque d'ostéosynthèse selon l'une des revendications 1 à 19, **caractérisée en ce qu'**il comprend en outre au moins une vis d'ostéosynthèse (11).

**21.** Dispositif de fixation selon la revendication 20, **caractérisé en ce que** la vis d'ostéosynthèse (11) comprend une structuration tridimensionnelle (12) réalisée sur la tête de vis (13), de préférence sous la forme d'un filet extérieur, et correspondant à la structuration tridimensionnelle (5).

**22.** Dispositif de fixation selon la revendication 20 ou 21, **caractérisé en ce que** la vis d'ostéosynthèse (11) est réalisée sous forme de vis autotaraudeuse et/ou autoforeuse.

Fig. 1

Fig. 2

Fig. 3

Fig. 4